# FASCICULE DE BREVET EUROPEEN

(11) **EP 0 763 136 B1**
(45) Date de publication et mention de la délivrance du brevet: **16.12.1998**
(21) Numéro de dépôt: 95921878.5
(22) Date de dépôt: 02.06.1995
(51) Int. Cl.: C12Q 1/68, C12N 15/12

(54) **PROCEDE ET SONDES POUR LA DETECTION DE MARQUEURS LIES AU LOCUS DES AMYOTROPHIES SPINALES INFANTILES**
VERFAHREN UND PROBEN ZUM NACHWEIS VON MIT DEM LOCUS DER INFANTILEN SPINALAMYOTROPHIEN VERBUNDENEN MARKERN
METHOD AND PROBES FOR DETECTING MARKERS BOUND TO THE LOCUS OF CHILD SPINAL MUSCULAR ATROPHIES

(30) Priorité: 03.06.1994 FR 9406856
(43) Date de publication de la demande: 19.03.1997
(73) Titulaire: INSTITUT NATIONAL DE LA SANTE ET DE LA RECHERCHE MEDICALE (INSERM), 75654 Paris Cédex 13 (FR)
(72) Inventeur: MELKI, Judith, F-75015 Paris (FR); MUNNICH, Arnold, F-75006 Paris (FR)
(74) Mandataire: Desaix, Anne
(86) Numéro de dépôt international: FR9500722
(87) Numéro de publication internationale: WO9533852

(56) Documents cités:
- WO-A-92/00386
- GENOMICS, vol. 20, SAN DIEGO US, page 84 WIRTH B ET AL 'Large linkage analysis in 100 families with autosomal recessive spinal muscular atrophy (SMA) and 11 CEPH families using 15 polymorphic loci in the region 5q11.2-q13.3.'
- AMERICAN JOURNAL OF HUMAN GENETICS, vol. 54, CHICAGO US, page 687 O.CLERMONT ET AL. 'Use of genetic...' cité dans la demande
- AM J HUM GENET, MAR 1994, 54 (3) P482-8, vol. 54, CHICAGO US, page 482 BRZUSTOWICZ LM ET AL 'Paternal isodisomy for chromosome 5 in a child with spinal muscular atrophy.'
- HUM MOL GENET, AUG 1993, 2 (8) P1169-76, vol. 2, OXFORD GB, page 1169 THOMPSON TG ET AL 'High resolution physical map of the region surrounding the spinal muscular atrophy gene.'
- PRENATAL DIAGNOSIS, vol. 13, COLCHESTER GB, page 643 HUSCHENBETT J ET AL 'Prenatal diagnosis of the acute form of proximal spinal muscular atrophy: experience on the acceptance of linkage analyses by the families'
- SCIENCE., vol. 264, LANCASTER, PA US, page 1474 MELKI J ET AL 'De novo and inherited deletions of the 5q13 region in spinal muscular atrophies.'

### LISTE DE SEQUENCES

### (1) INFORMATIONS GENERALES:

(i) DEPOSANT:
   (A) NOM: INSERM
   (B) RUE: 101, Rue de Tolbiac
   (C) VILLE: PARIS
   (E) PAYS: FRANCE
   (F) CODE POSTAL: F 75654
(ii) TITRE DE L' INVENTION: PROCEDE ET SONDES POUR LA DETECTION DE MARQUEURS LIES AU LOCUS DES AMYOTROPHIES SPINALES INFANTILES
(iii) NOMBRE DE SEQUENCES: 13
(iv) FORME DECHIFFRABLE PAR ORDINATEUR:
   (A) TYPE DE SUPPORT: Floppy disk
   (B) ORDINATEUR: IBM PC compatible
   (C) SYSTEME D'EXPLOITATION: PC-DOS/MS-DOS
   (D) LOGICIEL: PatentIn Release #1.0, Version #1.30 (OEB)
(v) DONNEES DE LA DEMANDE ACTUELLE:
   NUMERO DE LA DEMANDE: EP 95921878.5

### (2) INFORMATIONS POUR LA SEQ ID NO: 1:

(i) CARACTERISTIQUES DE LA SEQUENCE:
   (A) LONGUEUR: 372 paires de bases
   (B) TYPE: nucléotide
   (C) NOMBRE DE BRINS: double
   (D) CONFIGURATION: linéaire
(ii) TYPE DE MOLECULE: ADN (génomique)
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 1:
   ACCTGANCCC AGANGGTCAA GGCTGCAGTG AGACGAGATT GCNCCACTGC CCTCCACCCT    60
   GGGTGATAAG AGTGGGACCC TGTNTCAAAA CATACACACA CACACACACA CACACACACA   120
   CACACACACA CACACTCTCT CTCTCTCTCT CTCTCTCTCT CTCTCTCTCT CTCTCTCTCA   180
   AAAACACTTG GTCTGTTATT TTTNCGAAAT TGTCAGTCAT AGTTATCTGT TAGACCAAAG   240
   CTGNGTAAGN ACATTTATTA CATTGCCTCC TACAACTTCA TCAGCTAATG TATTTGCTAT   300
   ATAGCAATTA CATATNGGNA TATATTATCT TNAGGGGATG GCCANGTNAT AAAACTGTCA   360
   CTGAGGAAAG GA                                                       372

### (2) INFORMATIONS POUR LA SEQ ID NO: 2:

(i) CARACTERISTIQUES DE LA SEQUENCE:
   (A) LONGUEUR: 294 paires de bases
   (B) TYPE: nucléotide
   (C) NOMBRE DE BRINS: double
   (D) CONFIGURATION: linéaire
(ii) TYPE DE MOLECULE: ADN (génomique)
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 2:

### (2) INFORMATIONS POUR LA SEQ ID NO: 3:

(i) CARACTERISTIQUES DE LA SEQUENCE:
   (A) LONGUEUR: 141 paires de bases
   (B) TYPE: nucléotide
   (C) NOMBRE DE BRINS: double
   (D) CONFIGURATION: linéaire
(ii) TYPE DE MOLECULE: ADN (génomique)
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 3:
   TCGAGGTAGA TTTGTATTAT ATCCCATGTA CACACACACA CACACACACA CACACACACA    60
   CACACACAGA CTTAATCTGT TTACAGAAAT AAAAGGAATA AAATACCGTT TCTACTATAC   120
   ACCAAAACTA GCCATCTTGA C                                             141

### (2) INFORMATIONS POUR LA SEQ ID NO: 4:

(i) CARACTERISTIQUES DE LA SEQUENCE:
   (A) LONGUEUR: 305 paires de bases
   (B) TYPE: nucléotide
   (C) NOMBRE DE BRINS: double
   (D) CONFIGURATION: linéaire
(ii) TYPE DE MOLECULE: ADN (génomique)
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 4:
   CCCTGAGAAG GCTTCCTCCT GAGTATGCAT AAACATTCAC AGCTTGCATG CGTGTGTGTG    60
   TGTGTGTGTG TGTGTATGTT TGCTTGCACT GTAAAAACAA TTGCAACATC AACAGAAATA   120
   AAAATTAAAG GAATAATTCT CCTCCGACTC TGCCGTTCCA TCCAGTGAAA CTCTTCATTC   180
   TGGGGTAAAG TTCCTTCAGT TCTTTCATAG ATAGGTATAT ACTTCATAAG TCAAACAATC   240
   AGGCTGGGTG CAGTAGCTCA TGCCTGTAAT CCCAGCCCTT TGGGAGGCCG AGCTGGGCAG   300
   ATCGA                                                               305

### (2) INFORMATIONS POUR LA SEQ ID NO: 5:

(i) CARACTERISTIQUES DE LA SEQUENCE:
   (A) LONGUEUR: 350 paires de bases
   (B) TYPE: nucléotide
   (C) NOMBRE DE BRINS: double
   (D) CONFIGURATION: linéaire
(ii) TYPE DE MOLECULE: ADN (génomique)
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 5:
   TCCACCCGCC TTGGCCTCCC AAAGCNCTGG GATTACAGGC GTGACTGCCG CACCCAGCTG    60
   TAAACTGGNT TNNTAATGGT AGATTTTNAG GTATTAACAA TAGATAAAAA GATACTTTTN   120
   GGCATACTGT GTATTGGGAT GGGGTTAGAA CAGGTGTNCT ACCCAAGACA TTTACTTAAA   180
   ATCGCCCTCG AAATGCTATG TGAGCTGTGT GTGTGTGTGT GTGTGTGTGT GTATTAAGGA   240
   AAAGCATGAA AGTATTTATG CTTGATTTTT TTTTTTNACT CATAGCTTCA TAGTGGANCA   300
   GATACATAGT CTAAATCAAA ATGTTTAAAC TTTTTATGTC ACTTGCTGTC              350

### (2) INFORMATIONS POUR LA SEQ ID NO: 6:

(i) CARACTERISTIQUES DE LA SEQUENCE:
   (A) LONGUEUR: 20 paires de bases
   (B) TYPE: nucléotide
   (C) NOMBRE DE BRINS: simple
   (D) CONFIGURATION: linéaire
(ii) TYPE DE MOLECULE: ADN
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 6:
   CCTCCACCCT GGGTGATAAG    20

### (2) INFORMATIONS POUR LA SEQ ID NO: 7:

(i) CARACTERISTIQUES DE LA SEQUENCE:
   (A) LONGUEUR: 21 paires de bases
   (B) TYPE: nucléotide
   (C) NOMBRE DE BRINS: simple
   (D) CONFIGURATION: linéaire
(ii) TYPE DE MOLECULE: ADN
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 7:
   GCTGATGAAG TTGTAGGAGG C 21

### (2) INFORMATIONS POUR LA SEQ ID NO: 8:

(i) CARACTERISTIQUES DE LA SEQUENCE:
   (A) LONGUEUR: 20 paires de bases
   (B) TYPE: nucléotide
   (C) NOMBRE DE BRINS: simple
   (D) CONFIGURATION: linéaire
(ii) TYPE DE MOLECULE: ADN
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 8:
   TAGAGACGGG GTTTCGGCAT 20

### (2) INFORMATIONS POUR LA SEQ ID NO: 9:

(i) CARACTERISTIQUES DE LA SEQUENCE:
   (A) LONGUEUR: 19 paires de bases
   (B) TYPE: nucléotide
   (C) NOMBRE DE BRINS: simple
   (D) CONFIGURATION: linéaire
(ii) TYPE DE MOLECULE: ADN
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 9:
   GATCTGCCTT CCTTCCTGC 19

### (2) INFORMATIONS POUR LA SEQ ID NO: 10:

(i) CARACTERISTIQUES DE LA SEQUENCE:
   (A) LONGUEUR: 20 paires de bases
   (B) TYPE: nucléotide
   (C) NOMBRE DE BRINS: simple
   (D) CONFIGURATION: linéaire
(ii) TYPE DE MOLECULE: ADN
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 10:
   GGCTTCCTCC TGAGTATGCA 20

### (2) INFORMATIONS POUR LA SEQ ID NO: 11:

(i) CARACTERISTIQUES DE LA SEQUENCE:
   (A) LONGUEUR: 20 paires de bases
   (B) TYPE: nucléotide
   (C) NOMBRE DE BRINS: simple
   (D) CONFIGURATION: linéaire
(ii) TYPE DE MOLECULE: ADN
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 11:
   GTTTCACTGG ATGGAACGGC 20

### (2) INFORMATIONS POUR LA SEQ ID NO: 12:

(i) CARACTERISTIQUES DE LA SEQUENCE:
   (A) LONGUEUR: 20 paires de bases
   (B) TYPE: nucléotide
   (C) NOMBRE DE BRINS: simple
   (D) CONFIGURATION: linéaire
(ii) TYPE DE MOLECULE: ADN
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 12:
   ATCGCCCTCG AAATGCTATG 20

### (2) INFORMATIONS POUR LA SEQ ID NO: 13:

(i) CARACTERISTIQUES DE LA SEQUENCE:
   (A) LONGUEUR: 21 paires de bases
   (B) TYPE: nucléotide
   (C) NOMBRE DE BRINS: simple
   (D) CONFIGURATION: linéaire

(ii) TYPE DE MOLECULE: ADN
(iii) HYPOTHETIQUE: NON
(iv) ANTI-SENS: NON
(vi) ORIGINE:
   (A) ORGANISME: Homo sapiens
(xi) DESCRIPTION DE LA SEQUENCE: SEQ ID NO: 13:
   CTGTTCCACT ATGAAGCTAT G 21

## Description

Les amyotrophies spinales infantiles (SMA) représentent le groupe d'affections héréditaires récessives autosomiques fatales le plus fréquent après la mucoviscidose (incidence : 1/6 000) (1). Les SMAs sont caractérisées par une dégénérescence des motoneurones de la corne antérieure de la moelle épinière, entraînant une paralysie progressive des membres et du tronc associée à une atrophie musculaire. Les amyotrophies spinales infantiles sont divisées en catégories dites de types I, II et III, selon l'âge de début des symptômes et leur évolutivité (2).

Il est rappelé que les amyotrophies spinales de type I ne s'observent que chez le nouveau-né et chez le nourrisson : c'est le type le plus grave, l'espérance de vie de ces enfants ne dépassant guère quelques années.

Les amyotrophies spinales de type II se manifestent également dès la première année de vie des enfants atteints, mais après qu'ils aient acquis la station assise. Ils ne peuvent alors plus guère se lever et atteignent au mieux le stade de l'adolescence.

Enfin, l'amyotrophie spinale de type III apparaît chez l'enfant, lorsque celui-ci commence à marcher, les symptômes rappelés plus haut se développant alors plus lentement.

Hormis le diagnostic fondé sur des signes cliniques et paracliniques (électromyographie et biopsie musculaire) d'apparition de la maladie, signes qui ne présentent souvent pas la spécificité requise, il n'existe guère d'autres procédés permettant un diagnostic équivalent, fut-ce seulement dans un nombre limité de cas. Il va sans dire que le clinicien est encore plus désarmé, s'agissant d'anticiper l'apparition future de la maladie chez des patients présumés à risques ou, en médecine néonatale, chez le foetus.

L'anomalie biochimique de cette affection est inconnue. Néanmoins, le gène responsable des trois formes de SMA a été localisé, par analyse de liaison génétique, sur le chromosome 5q11.2-q13.3 (3-6), de sorte qu'il s'agirait d'affections alléliques.

Récemment, le gène responsable des SMAs a été localisé sur le chromosome 5 dans un intervalle de 2 centimorgans (cM) défini par les loci flanquants D5S629 et D5S637 (7).

Plusieurs fragments issus de ce chromosome ont été clonés dans des chromosomes artificiels de levures ou YACs (abréviation de l'expression anglaise correspondante : "Yeast Artificial Chromosomes"). Plusieurs contigs de YACs de la région 5q13 ont été décrits. l'expression "contig de la région 5q13" se rapporte à un ensemble de YACs dont les insérats formés de fragments de la région 5q13 peuvent être considérés comme se chevauchant les uns les autres, cet ensemble de fragments recouvrant néanmoins la totalité de la région 5q13. L'un de ces contigs était constitué de 7 YACs chevauchants couvrant approximativement 3,2 Mb (13), l'autre de 10 YACs chevauchants couvrant une région de 2 Mb (14). Mais aucun des marqueurs connus jusqu'à ce jour ne permet l'établissement d'une corrélation, fut elle seulement partielle mais fiable, entre la détection que l'on peut en faire et les signes cliniques de la maladie.

L'invention repose plus particulièrement sur la découverte que la région 5q13 comportait plusieurs loci qui, abstraction faite de séquences répétitives en nombre variable, possédaient des séquences de nucléotides sensiblement identiques et formaient autant de marqueurs polymorphes caractéristiques de cette région. Le polymorphisme de ces marqueurs est à la source de la capacité que l'on a de les discriminer, de les corréler parfois à ceux que portent les parents des sujets étudiés, ou au contraire, à identifier le caractère de novo des mutations ou délétions dont ils peuvent être l'objet. C'est également ce polymorphisme qui est à l'origine de la capacité dont l'on dispose désormais dans un certain nombre de cas, soit d'y trouver la confirmation du caractère correct d'un diagnostic clinique de SMA, notamment de type 1, soit parfois de l'exclure, par exemple en médecine néonatale et lorsqu'il existe un antécédent de la maladie chez un frère ou une soeur.

Ces nouveaux marqueurs d'ADN plus particulièrement des marqueurs polymorphes (microsatellites), ont été identifiés par criblage de fragments, contenus dans le susdit intervalle de deux cM identifié par (7) et contenu dans un YAC de 4 Mb. Ce criblage a été effectué en mettant en oeuvre une banque de YACs du Centre d'Etudes du Polymorphisme Humain (CEPH), Paris, France, et cela en utilisant les YACs de première génération 755B12 (contenant le marqueur AFM265wf5) et 751E3 (contenant le marqueur AFM 281yh9), par amplification PCR entre les amorces spécifiques de chaque marqueur selon la méthode tridimensionnelle (3d) précédemment décrite (8). Une marche chromosomique, selon une méthode préalablement décrite (12), a permis l'identification de nouveaux YACs chevauchant les YACs précédents, et contenant les nouveaux marqueurs d'ADN polymorphes (microsatellites).

Il a notamment été procédé comme suit.

### Familles

La carte génétique haute résolution préalablement effectuée avait permis de reconnaître des événements de recombinaison entre le locus SMA et les loci polymorphes flanquants les plus proches (D5S629 et D5S637) (7). L'analyse génétique des neuf familles comportant des recombinaisons clefs a été réalisée avec les nouveaux marqueurs polymorphes. Il s'agit soit de familles consanguines (n = 5) soit de familles comportant plusieurs sujets atteints (n = 4). Cette analyse génétique a consisté en la construction des haplotypes les plus vraisemblables, établis en minimisant le nombre d'événements de recombinaison méiotique.

### Criblage des banques de YAC

Les banques de YAC du CEPH ont été criblées par amplification PCR entre les amorces spécifiques de chaque marqueur selon la méthode tridimensionnelle (3d) précédemment décrite (8). Le génotype des YACs contenant les marqueurs microsatellites a été établi par électrophorèse des produits de PCR sur gel de polyacrylamide dénaturant, transféré sur membrane de nylon chargé et hybridé avec un oligonucléotide (CA)n ou (CT)n marqué au ³²P. La détection des répétitions dinucléotidiques a été effectuée par une méthode décrite (9). La taille des YACs a été estimée après électrophorèse en champ pulsé.

### Génération des marqueurs à partir des YACs sélectionnés

L'ADN des YACs a été séparé des chromosomes de levure après électrophorèse en champ pulsé sur gel d'agarose, connu sous la marque "SEAPLAQUE GTG 1 %". Après traitement par la b agarase, l'ADN a été précipité dans l'éthanol. Un total de 300 ng de YAC a été digéré par l'enzyme de restriction Sau 3A, puis réparé partiellement, avant d'être cloné dans le bactériophage M13 au site Sal 1 réparé partiellement.

Les clones M13 contenant des répétitions (CA) ou (CT) ont été détectés grâce au criblage par les oligonucléotides (CA) 20 ou (CT) 20 marqués au ³²P. Les matrices positives ont alors été séquencées (10). Des amorces oligonucléotidiques flanquant les répétitions (CA) ou (CT) ont été choisies. La localisation chromosomique des marqueurs a été déterminée après amplification PCR des ADNs d'un panel d'hybrides somatiques du chromosome 5. Ce panel comporte la lignée HHW105 contenant comme unique chromosome humain le chromosome 5 entier, la lignée HHW1064 contenant le chromosome 5 délété dans la région 5q11-q14 et la lignée HHW213 contenant le chromosome 5p (11). Les loci polymorphes ont été reconnus en testant 5 individus contrôles non apparentés. Les marqueurs non polymorphes ont été utilisés comme STS (abréviation de l'expression anglaise "Sequence Tagged Site" ou site de séquence marqué).

### Marche chromosomique

Cette marche a utilisé, à partir des YACs sélectionnés, les produits d'amplification PCR générés entre l'oligonucléotide A33 choisi dans les séquences moyennement répétées de type ALU. Ces produits ont permis la sélection de nouveaux YACs chevauchant les précédents selon la méthode préalablement décrite (12). Des nouveaux marqueurs d'ADN polymorphes ont été alors de nouveau isolés à partir de ces YACs.

Cette marche chromosomique a permis l'identification de nouveaux YACs chevauchant les précédents, et de nouveaux marqueurs d'ADN ont alors été isolés. Sur les 28 marqueurs identifiés, 9 ont été exclus de l'étude car ils étaient aussi présents sur le bras court du chromosome 5, démontrant l'existence d'une duplication partielle de la région 5q13 sur le chromosome 5p (tableau 1).

Les 19 marqueurs spécifiques de la région 5q13 ont été retenus pour sélectionner des YACs potentiellement chevauchants. Neuf d'entre eux détectent plus d'un locus dans la région 5q13 (tableau 1). En effet, quatre marqueurs microsatellites polymorphes [C212 (D5F149S1-S2), C271 (D5F148S1-S2), C272 (D5F150S1-S2), C171 (D5F151S1-S2)] ont révélé la présence de deux produits d'amplification et un marqueur [C161(D5F153S1-S2-S3)] a révélé 3 produits d'amplification sur l'hybride somatique HHW105. Aucun d'eux n'était localisé en dehors de la région 5q13. Ces résultats démontrent que les marqueurs C212, C272, C271, C171 détectent 2 loci et le marqueur C161 détecte trois loci dans la région 5q13. Ces résultats indiquent la présence d'éléments répétés sur le chromosome 5q13 (tableau 1).

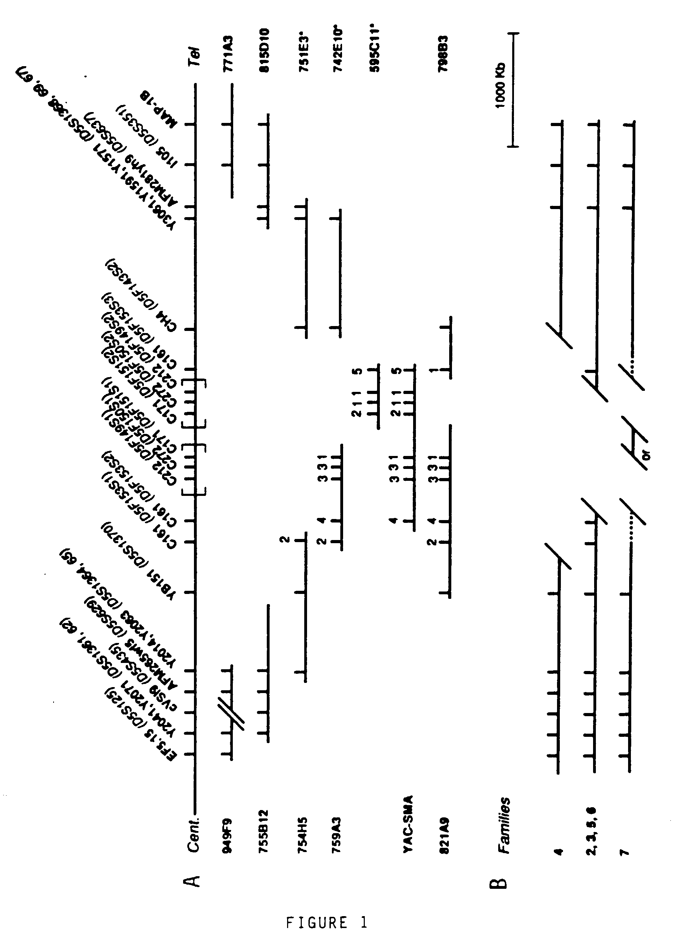

Du fait de l'organisation génomique complexe de cette région, ces loci polymorphes ont été utilisés pour sélectionner les YACs chevauchants et les génotyper par rapport à l'ADN du donneur, afin de s'assurer que toute la région était couverte. La confirmation de cette organisation génomique a été apportée par l'identification d'un YAC (903D1) dont le génotype est identique à ceux des YACs 595C11 et 759A3. Le contig de YAC contient un minimum de 5 YACs couvrant approximativement 4 Mégabases depuis le marqueur AFM265wf5 jusqu'au marqueur AFM281yh9 (figure 1).

### Cartographie génétique haute résolution au locus SMA

Basé sur l'analyse conventionnelle des haplotypes et sur la méthode d'homozygotie par la descendance, 10 méioses recombinantes clefs ont été sélectionnées. L'analyse génétique de ces méioses recombinantes a été réalisée avec les nouveaux marqueurs polymorphes isolés à partir du contig de YAC (figure 1). Les marqueurs les plus polymorphes C212 et C272 ne révèlent plus d'événement de recombinaison avec le locus SMA. Ces résultats démontrent que ces marqueurs détectent les loci les plus proches du gène des SMAs.

L'invention concerne plus particulièrement des moyens (acides nucléiques et procédés) permettant la détection et, s'il y a lieu, la discrimination de plusieurs de ces marqueurs polymorphes.

En particulier, elle concerne des brins d'ADN dont chacun est caractérisé en ce que sa propre séquence de nucléotides :
- est contenue dans la séquence nucléique de l'un des brins d'un marqueur polymorphe d'une région chromosomique humaine réputée comporter un locus SMA, ce marqueur contenant des séquences de répétition, notamment dinucléotidiques, en nombre variable ;
- contient elle-même une séquence caractéristique présente dans ce marqueur polymorphe mais extérieure à la région de celui-ci qui contient lesdites séquences de répétition, cette séquence caractéristique présentant une taille suffisante, notamment d'au moins 12 et, de préférence, d'au moins 15 nucléotides, pour qu'un fragment de même séquence soit utilisable en tant qu'amorce pour la détection par PCR de marqueurs correspondants dans une préparation d'ADN d'origine humaine.

Parmi les brins préférés de l'invention, on mentionnera ceux dont la propre séquence -ou la séquence qui lui est complémentaire- est constituée par tout ou partie de l'une des régions extérieures à la région contenant les séquences de répétition, respectivement contenues dans l'une des séquences d'ADNs ci-après identifiées sous les désignations C212, C272, AFM157, C161 et C171.

Ces brins d'ADNs, surtout s'ils sont assez longs, peuvent être utilisés comme sondes directes, par exemple pour la présence ou non d'une délétion dans le marqueur correspondant. L'invention concerne alors également dans ce cas des ADNs recombinants (simple brins ou double brins) contenant ce brin recombinant à un acide nucléique non humain et non susceptible d'hybrider avec un fragment d'ADN humain.

Cependant, de préférence, les brins préférés sont ceux qui peuvent être utilisés dans un procédé d'amplification de chaînes, notamment de type PCR (abréviation de l'expression anglaise "Polymerase Chain Reaction" ou réaction de chaînes en présence d'une polymérase).

En particulier, l'invention concerne les paires d'amorces comportant de préférence au moins 12, avantageusement au moins 15 nucléotides dont les séquences sont respectivement contenues dans les paires de séquences, notamment issues de :
- **C212** ou vice versa
- **C272** ou vice versa
- **AFM157xd10** ou vice versa
- **C161** ou vice versa
- **C171** ou vice versa
   étant naturellement entendu que l'expression "vice versa" signifie que chacune des amorces issues des paires susdites de séquences (1) et (2) pourrait être remplacée par une amorce complémentaire. A titre d'exemples de paires d'amorces préférées, correspondant aux familles sus-indiquées, on mentionne les oligo-nucléotides suivants :

L'invention concerne naturellement aussi des brins d'ADN qui peuvent être considérés comme des répliques entières des marqueurs correspondants, ces brins (qui comportent alors également les séquences répétées correspondantes), pouvant aussi être utilisées comme sondes spécifiques des marqueurs correspondants, et ce par exemple à des fins de vérification de l'existence ou non de ce marqueur spécifique sur l'un des allèles d'un chromosome étudié.

L'invention concerne aussi un procédé pour l'étude de la région chromosomique réputée contenir au moins l'un des gènes impliqués, soit par sa présence, soit par son absence, dans la SMA et provenant d'un sujet (patient ou foetus) atteint ou susceptible d'être atteint, ce procédé comprenant la mise en contact d'un échantillon d'ADN chromosomique de ce sujet ou foetus avec au moins l'un des brins d'ADN tel que définis ci-dessus, notamment dans des conditions amplificatrices autorisant, dès lors qu'il s'hybriderait à des marqueurs correspondants de cet ADN chromosomique, l'élongation de ses chaînes vers et au-delà des séquences répétées de ces marqueurs, puis à se placer dans des conditions permettant de discriminer entre les chaînes d'élongation obtenues selon leurs longueurs respectives, pour obtenir des informations représentatives du nombre de ces marqueurs.

Il sera de préférence procédé par PCR, l'échantillon d'ADN chromosomique étant alors mis en contact avec une paire d'amorces telles que ci-dessus définies, susceptibles de s'hybrider aux régions des marqueurs, extérieures à celles qui contiennent leurs séquences répétées respectives, la discrimination entre les chaînes d'élongation produites étant alors obtenues, notamment par leur migration dans un gel électrophorétique approprié.

Il résulte, notamment des expériences dont les résultats sont relatés plus loin, que dans un pourcentage appréciable de cas, l'existence ou la potentialité d'une SMA, notamment de type 1, se manifeste par un nombre de marqueurs polymorphes détectés inférieur à celui observé dans des ADNs chromosomiques provenant de sujets sains, cette réduction témoignant de ce que chez les sujets atteints, des parties essentielles de la région 5q13 ont en fait été délétées.

Dans des formes d'exécution préférées de l'invention, plus particulièrement appliquées à des sujets à risques, notamment en raison de l'existence présente ou passée d'autres cas de SMA dans la même famille, le procédé selon l'invention sera également et à titre comparatif mis en oeuvre sur des préparations chromosomiques provenant des parents ou autres personnes apparentées.

Les essais dont les résultats sont exposés ci-après, tendent à montrer que ceux des sujets ayant reçu de leurs deux parents un allèle contenant un marqueur ayant donné matière à élongation de chaîne, sont moins menacés par une SMA, notamment de type 1, que ceux qui n'en ont reçu qu'un ou dont l'un des allèles n'est par porteur d'un tel marqueur.

Du fait du polymorphisme des marqueurs mis en oeuvre, il est en effet possible de discerner, notamment grâce aux migrations différentiées des produits d'amplification, tenant à leurs tailles différentes, ceux des allèles porteurs d'un marqueur hérité, le cas échéant, de l'un des parents et ceux hérités, le cas échéant, de l'autre, ou encore, comme l'invention a permis de le constater, l'apparition de délétions de novo, avec pour conséquence le risque d'apparition de la maladie chez des sujets dont les parents ne témoignaient apparemment d'aucune prédisposition pour elles.

A l'inverse, la détection chez un sujet donné, notamment chez le foetus, de la présence d'au moins un marqueur polymorphe sur chacun des allèles du chromosome correspondant, notamment lorsque l'étude comparative permettra de conclure que le foetus a hérité ces marqueurs de ses deux parents respectifs et qu'il n'est pas prédisposé à la maladie. Il pourrait en être de même pour des enfants en bas âge, chaque fois que les signes cliniques qu'ils présentent conduisent le clinicien à s'interroger sur la possibilité qu'ils auraient à développer la maladie.

On comprend alors l'importance du résultat que l'invention permet d'obtenir, au moins dans un certain nombre de cas, ne serait-ce que pour s'assurer, en médecine néonatale, de l'absence de potentialité génétique de la maladie chez le foetus ou pour confirmer un diagnostic négatif chez l'enfant en bas âge, alors que certains signes cliniques semblaient présager du contraire.

L'invention et les résultats qu'elle permet d'obtenir sont encore illustrés de façon plus détaillée -et non limitative- dans ce qui suit. Il sera également fait référence aux dessins, et au tableau, dont l'interprétation peut être faite en se reportant aux légendes qui apparaissent à la fin de la présente description.

### FAMILLES ET METHODES

### FAMILLES

Deux-cent-une familles de SMA ont été étudiées. Celles-ci ont été sélectionnées selon les critères diagnostiques retenus par le Consortium International sur les SMA (15). Les sujets ayant une SMA confirmée ont été répartis en trois sous-groupes (type I, II ou III). Quatre-vingt-dix patients appartenaient au type I, 81 au type II et 30 au type III. Deux grandes familles de référence du CEPH, 22 familles saines composées des deux parents et d'un enfant, et 60 individus sains non-apparentés ont été utilisés comme contrôles. Pour les besoins de l'analyse statistique, il n'a été inclus dans l'étude qu'un seul enfant atteint par famille.

### METHODES

### Buvardage de Southern (Southern blot) :

L'ADN des différents individus a été extrait à partir de culots de leucocytes ou de lignées lymphoblastoïdes. Il a ensuite été digéré par différents enzymes de restriction puis mis à migrer sur gel d'agarose 0.8%, avant d'être transféré sur membrane de nylon chargé.

### Détection des polymorphismes de répétition dinucléotidique :

A partir des mêmes ADNs, une amplification PCR a été réalisée entre les amorces spécifiques des marqueurs C212 (D5F149S1 et S2), C272 (D5F150S1 et S2) et C161 (D5F153S1, S2 et S3). Les conditions d'amplification sont les suivantes: dénaturation à 94°C pendant 1 min., hybridation à 55°C pendant 1 min. et extension à 72°C pendant 1 min. durant 30 cycles. Les produits de PCR sont ensuite mis à migrer sur gel de polyacrylamide dénaturant, transférés sur une membrane de nylon chargée positivement qui est hybridée avec une sonde reconnaissant les répétitions de dinucléotides CA (20).

### Construction d'une banque de phages à partir du YAC 595C11

Après digestion partielle par l'enzyme Sau 3A, l'ADN du clone 595C11 a été déposé et mis à migrer sur gel d'agarose (connu sous la désignation "SEAPLAQUE GTG 0.5%"). Après migration, les fragments, d'une taille de 12 à 23 Kb, sont découpés, digérés par la b-agarase puis précipités dans l'éthanol. Après réparation partielle du site Sau 3A, l'ADN est sous-cloné au site Xho 1 réparé partiellement du bactériophage FIX II (Stratagene). Les clones lambda contenant les marqueurs C212 (L-51), C272 (L-51, L-132) et C171 (L-5, L-13) ont été utilisés comme sondes pour rechercher des polymorphismes de restriction (RFLP) avec les enzymes de restriction EcoR I, Bgl II, Hind III et Xba I. La sonde JK53 (D5S112) a été utilisé comme contrôle interne pour les analyses de dosage génique. Les autoradiographies ont été examinées par densitométrie à 600 nm (Hoefer Scientific Instruments, San Francisco).

### RESULTATS

Pour tester l'hypothèse selon laquelle la présence d'éléments répétés pouvait favoriser la survenue de réarrangements chez les patients, la ségrégation des allèles a été analysée aux loci détectés par les marqueurs C212 et C272 chez 201 familles de SMA non consanguines. Une non-contribution parentale à ces loci a été observée chez huit enfants atteints appartenant à sept familles non apparentées. Quatre patients étaient atteints de la forme de type I, deux de type II et deux de type III. L'absence de contribution parentale était d'origine maternelle dans cinq des cas (familles 3, 4, 5, 6) et d'origine paternelle dans les trois autres (familles 1, 2, 7; figure 1). Un des patients de type I présentait un réarrangement survenu de novo n'emportant qu'un seul des deux loci reconnus par les marqueurs C212 et C272 (figure 1, famille 7). Des résultats identiques ont été obtenus en utilisant des paires d'oligonucléotides différentes pour chacun des marqueurs. La construction des haplotypes à l'aide des marqueurs flanquant le locus SMA (21) a permis d'exclure l'hypothèse d'une fausse paternité ou d'une erreur d'échantillonage. Ces résultats indiquent la présence de réarrangements emportant le locus morbide chez ces huit patients.

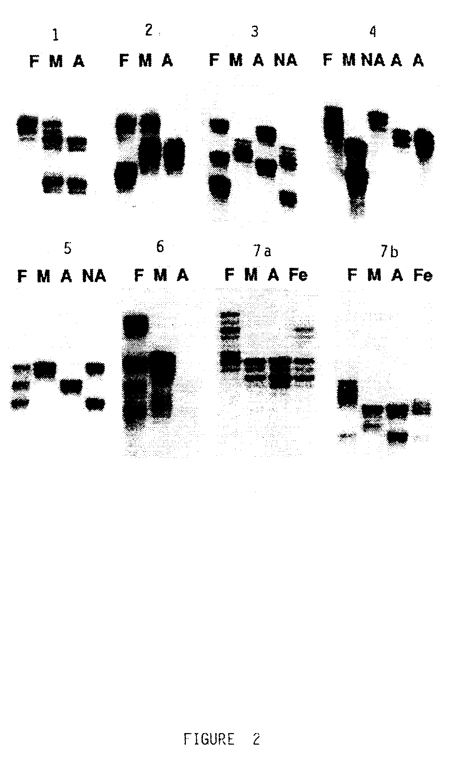

De plus, parce que le nombre de patients présentant un réarrangement avait pu être sous-estimé par un défaut d'informativité des méioses, le nombre de produits d'amplification PCR distincts révélés par les marqueurs C212 et C272 a été analysé chez les familles de SMA des trois types ainsi que chez 60 contrôles composés d'individus non-apparentés (figure 2). Chez 16 des 90 patients de type I (18%), un seul produit d'amplification PCR avec le marqueur C272 a été détecté, résultat statistiquement très différent de celui observé chez les patients de type II (1/81 soit 1%), de type III (1/30 soit 3%) et les contrôles (0/59 soit 0%, p<0.001). De même, la proportion de patients de type I présentant deux produits d'amplification PCR (55/90 soit 61%) est également significativement différente de celle des parents (69/180 soit 38%) et des contrôles (12/59 soit 20%, p<0.001). Des résultats similaires ont été obtenus avec le marqueur C212 (tableau). La possibilité que la réduction du nombre de fragments amplifiés chez les types I pouvait être due à une consanguinité éloignée a été exclue par comparaison du nombre de fragments partagés par les parents des sujets atteints de SMA et celui des parents de 20 familles non- apparentées contrôles. Les résultats n'ont alors pas montré de différence statistiquement significative (données non présentées).

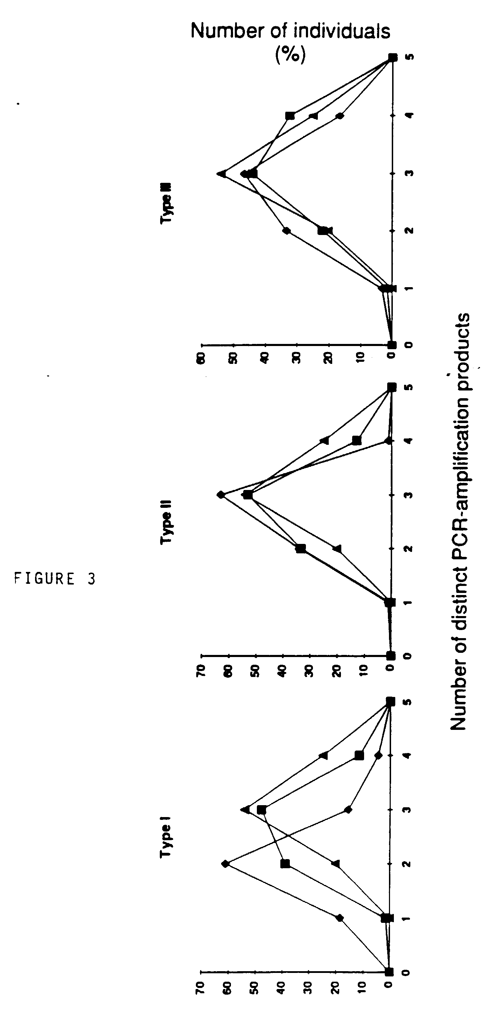

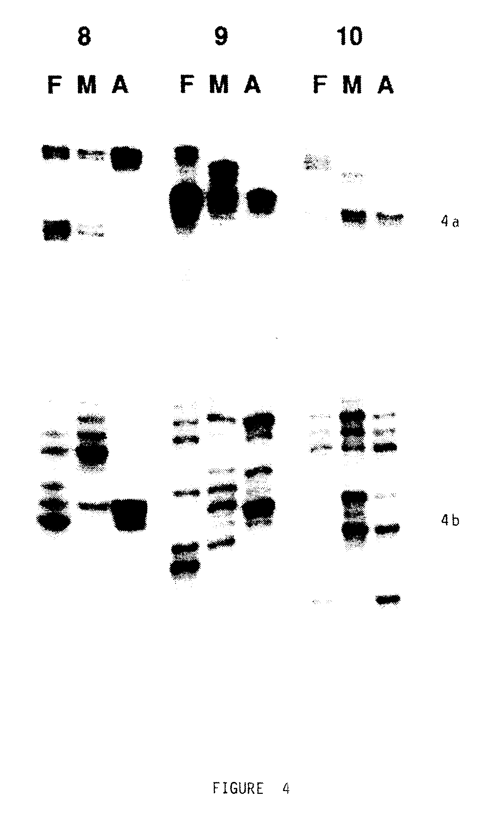

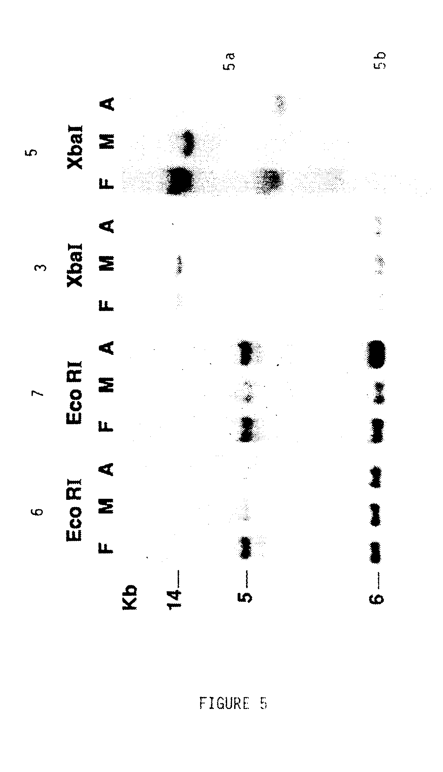

On a ensuite analysé avec le marqueur C161 les 20 familles de type I dont l'enfant atteint ne présentait qu'un produit d'amplification PCR avec les marqueurs C212 ou C272. Dans deux des vingt familles, nous avons observé un réarrangement survenant de novo et emportant 2 des 3 loci détectés par le marqueur C161 (figure 3, familles 8 et 9). Ces résultats soutiennent bien l'hypothèse selon laquelle la réduction allelique aux loci détectés par les marqueurs C212 et C272 chez les SMA de type I est due à une perte d'allèle à ces loci. Pour caractériser l'extension de la région 5q13 délétée chez ces patients, on a analysé ces familles avec les autres marqueurs polymorphes générés à partir du nouveau contig de YACs. L'haplotypage complet de ces familles nous a permis de cartographier les différentes délétions et de délimiter les bornes du plus petit remaniement entre les marqueurs C161 et C212-C272 (figure 4). Ces résultats suggèrent que le locus SMA se situe dans une région de 1.2 Mb contenue dans le YAC 903D1 (20, 22). Cependant, à cause de la parfaite homologie de séquence des régions flanquant les répétitions CA des marqueurs C212 et C272, il n'a pas été possible de déterminer lequel des deux loci manquait chez l'unique patient présentant la perte d'un seul locus (famille 7, figure 1 et 4). Pour fournir la preuve physique de la présence de délétions dans la région 5q13, le clone L-132, qui contient le marqueur C272, a été utilisé comme sonde pour l'analyse en Southern blot des familles de SMA montrant une contribution parentale anormale. L'analyse des RFLP et du dosage génique confirma alors la présence de délétions héritées ou de novo (figure 5).

### DISCUSSION ET CONCLUSION

La présente étude fournit la preuve génétique et physique directe de l'existence de délétions englobant le locus de la maladie chez 10 enfants atteints appartenant à 9 familles non-apparentées. De plus, la présence de délétions de la région 5q13 est fortement soutenue par l'observation d'une réduction allelique statistiquement associée à la forme sévère de la maladie (type I). Les délétions sont aussi rencontrées occasionnellement chez des patients de type II ou III. Des mutations alleliques distinctes pourraient donc expliquer l'expression clinique variable de la maladie. Ces résultats confirment aussi que le ou les gènes responsables des trois types de SMA se situent bien dans la même région. Les dystrophies musculaires de Duchenne ou de Becker peuvent également résulter de délétions mais, dans ce cas, il existe une différence mécanistique entre les types de délétions (24) alors qu'une telle analyse n'est pas encore rendue possible dans les SMA. Cette étude démontre également que les délétions peuvent se produire de novo, une caractéristique qui pourrait expliquer le faible coefficient de ségrégation précédemment rapporté par plusieurs auteurs dans les SMA (25, 26) et jusqu'ici assez mal compris. Les délétions de novo de la région 5q13 peuvent également expliquer l'apparente hétérogénéité génétique des SMA (18, 27) quand, dans une même famille, un enfant sain présente le même haplotype que l'enfant atteint avec les marqueurs flanquants. Cette observation est également importante pour le conseil génétique. En effet, l'apparente haplo-identité entre un foetus et l'enfant atteint d'une même famille déterminée à l'aide des marqueurs flanquants peut conduire à des erreurs de diagnostic prénatal lorsqu'une délétion de novo survient. La présence d'éléments répétés dans la région 5q13 peut expliquer l'instabilité de cette région contribuant ainsi à l'apparition de délétions chez les sujets atteints de SMA par des événements de crossing-over inégaux.

Il est à remarquer que la séquence C272 est apparemment contenue dans un fragment d'ADN (dénommé L132) susceptible de révéler la présence, soit de délétions héritées, soit de délétions de novo par l'analyse en "Southern blot" des polymorphismes de longueurs des fragments de restriction ou par l'analyse du dosage génique (dosage d'un certain taux de détérioration d'un gène du fait de l'affaiblissement d'un signal d'hybridation donnée par une sonde susceptible de le reconnaître).

**Tableau**

| **Marqueurs Statut** | **Type(n) Nombre** | | **produits P C R** d'amplifications distinctes | | | |
|---|---|---|---|---|---|---|
| | **d** | **e** | | | | |
| 1 | 2 | | 3 | 4 | | |
| C212 | I | | 13% | 13% | | |
| Atteints | (90) | | 66.5% | 6.5% | | |
| I I | | 2.5% | 35.5% | 59% | 2.5% | |
| (76) | | | | | | |
| I I I | | 3% | 23% | 66.5% | 6.5% | |
| (30) | | | | | | |
| Parents | I | | 1.5% | 42% | 45.5% | 10.5% |
| | (180) | | | | | |
| I I | | .5% | 30% | 53% | 16% | |
| (150) | | | | | | |
| I I I | 0 | | 23.5% | 52.5% | 23.5% | |
| (59) | | | | | | |
| Contrôles | (60) | | 0 | 18% | 60% | 21.5% |
| C272 | I | | 18.5% | 15.5% | | |
| Atteints | (90) | | 61% | 4.5% | | |
| I I | 1% | | 34% | 63% | 1% | |
| (81) | | | | | | |
| I I I | 3% | | 33% | 46.5% | 16.5% | |
| (30) | | | | | | |
| Parents | I | | 1.5% | 38.5% | 47.5% | 11.5% |
| | (180) | | | | | |
| I I | | .5% | 33% | 53% | 13% | |
| (162) | | | | | | |
| I I I | | 1.5% | 22% | 44% | 32% | |
| (59) | | | | | | |
| Contrôles | (59) | | 0 | 20% | 54% | 25% |
| Tableau. Nombre de produits d'amplification PCR distincts observé avec les marqueurs C212 et C272 chez les sujets atteints de SMA de type I, II et III, leurs parents et des contrôles. (n) indique le nombre d'individus testés. | | | | | | |

### LEGENDES DES FIGURES

**Figure 1** : Carte génétique des délétions chez les patients atteints de SMA.
**A**). Carte génomique de la région 5q13. Les différents marqueurs sont indiqués au-dessus de leur position génétique. Les parenthèses encadrant les blocs C212-C272-C171 indiquent que l'ordre des marqueurs à l'intérieur du bloc est inconnu.
**B**). Carte génétique des délétions. Les tirets correspondent aux marqueurs cités ci-dessus.

Dans la famille 7, la délétion peut entraîner soit le bloc centromèrique C212-C272-C171 soit le bloc télomèrique.

**Figure 2** : Contribution des allèles parentaux chez les patients atteints de SMA déterminé par les marqueurs C212 et C272. La figure montre l'étude des familles soit avec le marqueur C272 (familles 1, 2, 5 et 7b) soit avec le marqueur C212 (familles 3, 4, 6 et 7a). Les patients sont atteints de la forme de type I (familles 3, 5, 6 et 7), de type II (familles 1 et 2) ou de type III (famille 4). La construction des haplotypes avec les marqueurs flanquant le locus SMA (11) nous a permis de déterminer que les enfants sains des familles 4 et 5 avaient reçu l'allèle muté de leur mère. Dans la famille 7, on note la contribution incomplète du père à son enfant atteint avec les marqueurs C212 (7a) et C272 (7b) comparé au foetus haplo-identique comme établi avec les marqueurs flanquants (données non présentées). F: father (père), M: mother (mère), A: affected (atteint), NA: nonaffected (non-atteint), Fe: fetus (foetus). Les points indiquent les fragments alleliques.

**Figure 3** : Nombre de produits d'amplification PCR distincts révélés par le marqueur C272 chez les patients, leurs parents et des contrôles.

Un total de 90 patients de type I, 81 patients de type II, 30 patients de type III, leurs parents et 59 contrôles ont été étudiés. En abscisse figure le nombre de produits d'amplication PCR distincts, en ordonnée le nombre d'individus testés (%). atteints, : parents, : contrôles.

**Figure 4** : Preuve de l'existence de délétions survenues de novo détectées par le microsatellite C161 chez des patients atteints de type I.
**4a**. La ségrégation des allèles détectés par le marqueur C272 ne prouve ni n'exclue l'existence de délétions dans les familles 8, 9 et 10. On note que les patients ne présentent qu'un seul produit d'amplification PCR.
**4b.** La ségrégation des allèles détectés par le marqueur C161 révèle l'existence de délétions survenues de novo dans les familles 8 et 9 car l'enfant atteint n'a reçu qu'un seul locus de ses parents (famille 8) ou de son père (famille 9). Dans la famille 10, la détection de six produits d'amplification PCR distincts exclue toute possibilité d'une délétion emportant un des trois loci détectés par C161.
F: father (père), M: mother (mère), A: affected (atteint). Les points indiquent les fragments alleliques.

**Figure 5**: Analyse du polymorphisme de longueur des fragments de restriction (RFLP) et du dosage génique au locus détecté par le phage L-132 chez les patients atteints d'une SMA de type I. L'analyse génétique a été réalisée après digestion de l'ADN soit avec EcoR I (familles 6 et 7) soit avec Xba I (familles 3 et 5). Les membranes ont été hybridées avec le clone L-132 **5a** et la sonde JK53 **5b.** Le dosage génique a été déterminé par examen densitométrique du signal d'hybridation pour les familles 3, 6 et 7. Dans la famille 6, l'intensité de la bande est réduite de 50% chez la mère et l'enfant atteint ce qui indique qu'il s'agit d'une délétion héritée. Dans les familles 7 et 3, l'intensité de la bande des enfants atteints est nettement plus faible que celle des parents suggérant que la délétion soit survenue de novo. Dans la famille 5, l'analyse du RFLP détecté par l'enzyme Xba I montre que l'enfant atteint n'a pas reçu d'allèle de sa mère.

### REFERENCES

1. Pearn J. 1980. Classification of spinal muscular atrophies. Lancet; 1:919-922.
2. Munsat TL. 1991. Workshop report. International SMA collaboration. Neuromuscular Disorders; 1:81.
3. Brzustowicz LM, Lehner T, Castilla LH, Penchaszadeh GK, Wilhelmsen KC, Daniels R et al. 1990. Genetic mapping of chronic childhood-onset spinal muscular atrophy to chromosome 5q11.2-q13.3. Nature; 344:540-541.
4. Melki J, Abdelhak S, Sheth P, Bachelot MF, Burlet P, Marcadet A et al. 1990. Gene for chronic proximal spinal muscular atrophies maps to chromosome 5q. Nature; 344:764- 768.
5. Gilliam TC, Brzustowicz LM, Castilla LM, Lehner T, Penchaszadeh GK, Daniels RJ et al. 1990. Genetic homogeneity between acute and chronic forms of spinal muscular atrophy. Nature; 345:823-825.
6. Melki J, Sheth P, Abdelhak S, Burlet P, Bachelot MF, Lathrop GM et al. 1990. Mapping of acute (type I) spinal muscular atrophy to chromosome 5q12-q14. Lancet; 336:271- 273.
7. Clermont O, Burlet P, Burglen L, Lefebvre S, Pascal F, Weissenbach J et al. 1994. Use of genetic and physical mapping to locate the spinal muscular atrophy locus between two new highly polymorphic DNA markers. Am J Hum Genet. ; 54:687-694.
8. Green E.D and Olson M.V. 1990. Proc. Natl. Acad. Sci. USA. 87,213.
9. Hazan J, Dubay C, Pankowiak MP, Becuwe N, Weissenbach J. 1992. A genetic linkage map of human chromosome 20 composed entirely of microsatellite markers. Genomics; 12:183-189.
10. Smith LM, Sanders JZ, Kaiser RJ, Hughes P, Dodd C, Connell CR et al. 1986. Fluorescence detection in automated DNA sequence analysis. Nature; 321:674-679.
11. Carlock L.R, Sharecky D., Dana S. 1985. Am. J. Hum. Genet. 37.839.
.12. Chumakov IM, Le Gall I, Billault A, Ougen P, Soularue P, Guillou S et al. 1992. Isolation of chromosome 21-specific yeast artificial chromosomes from a total human genome library. Nature Genetics; 1:222-226.
13. Klein P.W, Wang C.H, Lyndon L.L, Vitale E, Pan J et al. 1993. Construction of a yeast artificial chromosome contig spanning the spinal muscular atrophy disease gene region. Proc. Natl. Acad. Sci. USA; 90:6801-6805.
14. Francis M.J, Morrison K.E, Campbell L, Grewal P.K, Christodoulou Z et al. 1993. A contig of non-chimaeric YACs containing the spinal muscular atrophy gene in 5q13.Human Molecular Genetics. 8:1161-1167.
15. Munsat T.L. (1991). Workshop report. International SMA collaboration. Neuromuscular Disorders 1, 81
16. Brzustowicz L.M., Lehner T., Castilla L.H., Penchaszadeh G.K., Wilhelmsen K.C., Daniels R. et al. (1990). Genetic mapping of chronic childhood-onset spinal muscular atrophy to chromosome 5q11.2-q13.3. Nature 344, 540-541
17. Melki J., Abdelhak S., Sheth P., Bachelot M.F., Burlet P., Marcadet A. et al. (1990). Gene for proximal spinal muscular atrophies maps to chromosome 5q. Nature 344, 764-768
18. Gilliam T.C., Brzustowicz L.M., Castilla L.H., Lehner T., Penchaszadeh G.K., Daniels R. et al. (1990). Genetic homogeneity between acute and chronic forms of spinal muscular atrophy. Nature 345, 823-825
19. Melki J., Sheth P., Abdelhak S., Burlet P., Bachelot M.F., Lathrop G.M. et al. (1990). Mapping of acute (type I) spinal muscular atrophy to chromosome 5q12-q14. Lancet 336, 271-273
20. Hazan J., Dubay C., Pankowiak M.P., Becuwe N. et Weissenbach J. (1992). A genetic linkage map of human chromosome 20 composed entirely of microsatellite markers. Genomics 12, 183-189
21. Clermont O., Burlet P., Bürglen L., Lefebvre S., Pascal F., Mc Pherson J., Wasmuth J.J., Cohen D., Le Paslier D., Weissenbach J., Lathrop M., Munnich A. et Melki J. (1994). Use of genetic and physical mapping to locate the spinal muscular atrophy locus between two new highly polymorphic DNA markers. Am. J. Hum. Genet 54,687-694
22. Melki J., Lefebvre S., Bürglen L., Burlet P., Clermont O., Millasseau P., Reboullet S., Bénichou B., Zeviani M., Le Paslier D., Cohen D., Weissenbach J. et Munnich A. (1994). De novo and inherited deletions of the 5q13 region in spinal muscular atrophies. Science, 264, 1474-1477.
23. Carlock L.R., Skarecky D., Dana S.L. et Wasmuth J.J. (1985). Deletion mapping of human chromosome 5 using chromosome-specific DNA probes. Am. J. Hum. Genet 37,839-852
24. Monaco A.P., Bertelson C.J., Liechti-Gallati S., Moser H. et Kunkel L.M. (1988). An explanation for the phenotypic differences between patients bearing partial deletions of the DMD locus. Genomics 2,90-95
25. Hausmanowa-Petrusewicz I., Zaremba I. et Borkowska J. (1985). Chronic proximal spinal muscular atrophy of childhood and adolescence: problems of classification and genetic counselling. J. Med. Genet 22,350-353
26. Zerres K. (1988). Genetik spinaler muskelatrophien. Habilitationsschrift Universität Bonn.
27. Daniels R.J., Tomas N.H., MacKinnon R.N., Lehner T., Ott J., Flint T.J. et al. (1992). Linkage analysis of spinal muscular atrophy. Genomics 12, 335-339

## Revendications

1. Brin d'ADN caractérisé en ce que sa propre séquence de nucléotides :
- est contenue dans la séquence nucléique de l'un des brins d'un marqueur polymorphe de la région chromosomique humaine 5q13 comportant un locus SMA et contenant des séquences de répétitions, notamment dinucléotidiques, en nombre variable ;
- contient elle-même une séquence caractéristique présente dans ce marqueur polymorphe mais extérieure à la région de celui-ci qui contient lesdites séquences de répétition, cette séquence caractéristique présentant une taille suffisante, notamment d'au moins 12 et, de préférence, d'au moins 20 nucléotides, pour qu'un fragment de même séquence soit utilisable en tant qu'amorce pour la détection par PCR de marqueurs correspondants dans une préparation d'ADN d'origine humaine.

2. Brin d'ADN selon la revendication 1, caractérisé en ce qu'une partie au moins de sa propre séquence, ou de la séquence qui lui est complémentaire, est contenue dans une région extérieure à la région contenant les répétitions de dinucléotides de cette dernière, au sein de la séquence **C212** qui suit (SEQ ID NO.1) :

3. Brin d'ADN selon la revendication 1, caractérisé en ce qu'une partie au moins de sa propre séquence, ou de la séquence qui lui est complémentaire, est contenue dans une région extérieure à la région contenant les répétitions de dinucléotides de cette dernière, au sein de la séquence **C272** qui suit (SEQ ID NO. 2) :

4. Brin d'ADN selon la revendication 1, caractérisé en ce qu'une partie au moins de sa propre séquence, ou de la séquence qui lui est complémentaire, est contenue dans une région extérieure à la région contenant les répétitions de dinucléotides de cette dernière, au sein de la séquence **AFM157xd10** qui suit (SEQ ID NO. 3) :

5. Brin d'ADN selon la revendication 1, caractérisé en ce qu'une partie au moins de sa propre séquence, ou de la séquence qui lui est complémentaire, est contenue dans une région extérieure à la région contenant les répétitions de dinucléotides de cette dernière, au sein de la séquence **C161** qui suit (SEQ ID NO. 4) :

6. Brin d'ADN selon la revendication 1, caractérisé en ce qu'une partie au moins de sa propre séquence, ou de la séquence qui lui est complémentaire, est contenue dans une région extérieure à la région contenant les répétitions de dinucléotides de cette dernière, au sein de la séquence **C171** qui suit (SEQ ID NO. 5) :

7. Paire d'amorces, notamment pour PCR, caractérisée en ce que leurs propres séquences sont contenues dans les deux séquences qui suivent : ou vice versa

8. Paire d'amorces selon la revendication 7, caractérisée en ce que les deux amorces présentent respectivement les séquences de nucléotides qui suivent :

9. Paire d'amorces, notamment pour PCR, caractérisée en ce que leurs propres séquences sont contenues dans les deux séquences qui suivent : ou vice versa

10. Paire d'amorces selon la revendication 9, caractérisée en ce que les deux amorces présentent respectivement les séquences de nucléotides qui suivent :

11. Paire d'amorces, notamment pour PCR, caractérisée en ce que leurs propres séquences sont contenues dans les deux séquences qui suivent : ou vice versa

12. Paire d'amorces selon la revendication 11, caractérisée en ce que les deux amorces présentent respectivement les séquences de nucléotides qui suivent :

13. Paire d'amorces, notamment pour PCR : ou vice versa

14. Paire d'amorces selon la revendication 13, caractérisée en ce que les deux amorces présentent respectivement les séquences de nucléotides qui suivent :

15. Paire d'amorces, notamment pour PCR, caractérisée en ce que leurs propres séquences sont contenues dans les deux séquences qui suivent :
**AFM157xd10**

16. Fragment d'ADNs caractérisé en ce que leurs séquences sont choisies parmi les séquences complètes définies dans l'une quelconque des revendications 2 à 6 ou les séquences qui leur sont respectivement complémentaires.

17. Fragment recombinant le fragment d'ADN de la revendication 16 ou d'une partie de ce fragment avec un fragment non humain.

18. Utilisation d'un fragment selon la revendication 17 en tant que sonde d'hybridation pour la détection directe du marqueur correspondant à ce fragment ou de la région le contenant.

19. Procédé pour l'étude de la région chromosomique humaine 5q13 contenant au moins l'un des gènes impliqués, soit par sa présence, soit par son absence, dans la SMA et provenant d'un sujet (patient ou foetus) atteint ou susceptible d'être atteint, ce procédé comprenant la mise en contact d'un échantillon d'ADN chromosomique de ce sujet ou foetus avec au moins l'un des brins d'ADN selon l'une des revendications 1 à 17, notamment dans des conditions amplificatrices autorisant, dès lors qu'il s'hybriderait à des marqueurs correspondants de cet ADN chromosomique, l'élongation de ses chaînes vers et au-delà des séquences répétées de ces marqueurs, puis à se placer dans des conditions permettant de discriminer entre les chaînes d'élongation obtenues selon leurs longueurs respectives, pour obtenir des informations représentatives du nombre de ces marqueurs.

20. Procédé selon la revendication 19, caractérisé en ce qu'il comprend la mise en contact de l'échantillon chromosomique avec une paire d'amorces conformes à l'une quelconque des revendications 7 à 15 et en ce que la discrimination entre les chaînes d'élongation est obtenue, notamment par migrations électrophorétiques sur gel.

## Claims

1. DNA strand, characterized in that its own nucleotide sequence:
- is contained in the nucleic acid sequence of one of the strands of a polymorphic marker of human chromosomal region 5q13 containing an SMA locus and containing a variable number of repeat sequences, in particular of dinucleotide repeats;
- itself contains a characteristic sequence present in this polymorphic marker but outside the region of the latter which contains said repeat sequences, this characteristic sequence being of sufficient size, in particular of at least 12 and preferably at least 20 nucleotides, for a fragment of the same sequence to be useable as primer for the detection by PCR of corresponding markers in a DNA preparation of human origin.

2. DNA strand according to claim 1, characterized in that at least a portion of its own sequence, or of the sequence which is complementary thereto, is contained in a region outside the region containing the dinucleotide repeats of the latter, within the sequence

3. DNA strand according to claim 1, characterized in that at least a portion of its own sequence, or of the sequence which is complementary thereto, is contained in a region outside the region containing the dinucleotide repeats of the latter, within the sequence

4. DNA strand according to claim 1, characterized in that at least a portion of its own sequence, or of the sequence which is complementary thereto, is contained in a region outside the region containing the dinucleotide repeats of the latter, within the sequence

5. DNA strand according to claim 1, characterized in that at least a portion of its own sequence, or of the sequence which is complementary thereto, is contained in a region outside the region containing the dinucleotide repeats of the latter, within the sequence

6. DNA strand according to claim 1, characterized in that at least a portion of its own sequence, or of the sequence which is complementary thereto, is contained in a region outside the region containing the dinucleotide repeats of the latter, within the sequence

7. Primer pair, in particular for PCR, characterized in that their own sequences are contained in the two sequences which follow: or vice versa

8. Primer pair according to claim 7, characterized in that the two primers possess, respectively, the nucleotide sequences which follow:

9. Primer pair, in particular for PCR, characterized in that their own sequences are contained in the two sequences which follow:
in the two sequences which follow: or vice versa

10. Primer pair according to claim 9, characterized in that the two primers possess, respectively, the nucleotide sequences which follow:

11. Primer pair, in particular of PCR, characterized in that their own sequences are contained in the two sequences which follow: or vice versa

12. Primer pair according to claim 11, characterized in that the two primers possess, respectively, the nucleotide sequences which follow:

13. Primer pair, in particular for PCR: or vice versa

14. Primer pair according to claim 13, characterized in that the two primers possess, respectively, the nucleotide sequences which follow:

15. Primer pair, in particular for PCR, characterized in that their own sequences are contained in the two sequences which follow:

16. Fragment of DNAs, characterized in that their sequences are chosen from the complete sequences defined in any one of claims 2 to 6 or the sequences which are, respectively, complementary thereto.

17. Fragment recombining the DNA fragment of claim 16 or of ' a portion of this fragment with a non-human fragment.

18. Use of a fragment according to claim 17 as hybridization probe for the direct detection of the marker corresponding to this fragment or of the region containing it.

19. Method for studying the 5q13 human chromosomal region which contains at least one of the genes involved, either by its presence or by its absence, in SMA and originating from a subject (patient or fetus) affected or liable to be affected, this method comprising bringing a chromosomal DNA sample of this subject or fetus into contact with at least one of the DNA strands of any one of claims 1 to 17, in particular under amplification conditions permitting, provided it would hybridize with corresponding markers of this chromosomal DNA, the elongation of its chains towards and beyond the repeat sequences of these markers, and then implementing conditions enabling a distinction to be made between the elongation chains obtained according to their respective lengths in order to obtain data representative of the number of these markers.

20. Method according to claim 19, characterized in that it comprises bringing the chromosomal sample into contact with a primer pair according to any one of claims 7 to 15, and in that the elongation chains are distinguished from one another, in particular by electrophoretic migrations on gel.

## Patentansprüche

1. DNA-Strang, dadurch gekennzeichnet, daß dessen eigene Nukleotidsequenz:
- in der Nukleinsequenz von einem der Stränge eines polymorphen Markers des menschlichen Chromosomenbereichs 5q13, der einen SMA-Locus umfaßt und repetitive Sequenzen, insbesondere Dinukleotidsequenzen, in variabler Anzahl enthält, enthalten ist,
- selbst eine charakteristische Sequenz enthält, die in diesem polymorphen Marker vorliegt, jedoch außerhalb von dessen Bereich, der die genannten repetitiven Sequenzen enthält, wobei diese charakteristische Sequenz eine ausreichende Größe, insbesondere von mindestens 12 und vorzugsweise mindestens 20 Nukleotiden aufweist, damit ein Fragment dieser Sequenz als Primer für den PCR-Nachweis entsprechender Marker in einer DNA-Präparation menschlichen Ursprungs eingesetzt werden kann.

2. DNA-Strang nach Anspruch 1, dadurch gekennzeichnet, daß mindestens ein Teil von dessen eigener Sequenz oder der Sequenz, die dazu komplementär ist, in einem Bereich außerhalb des Bereichs, der die Dinukleotid-Wiederholungen oder -Repeats dieses letzteren enthält, innerhalb der folgenden Sequenz C212 (SEQ ID NO. 1) enthalten ist:

3. DNA-Strang nach Anspruch 1, dadurch gekennzeichnet, daß mindestens ein Teil von dessen eigener Sequenz oder der Sequenz, die dazu komplementär ist, in einem Bereich außerhalb des Bereichs, der die Dinukleotid-Wiederholungen oder -Repeats dieses letzteren enthält, innerhalb der folgenden Sequenz C272 (SEQ ID NO. 2) enthalten ist:

4. DNA-Strang nach Anspruch 1, dadurch gekennzeichnet, daß mindestens ein Teil von dessen eigener Sequenz oder der Sequenz, die dazu komplementär ist, in einem Bereich außerhalb des Bereichs, der die Dinukleotid-Wiederholungen oder -Repeats dieses letzteren enthält, innerhalb der folgenden Sequenz **AFM157xd10** (SEQ ID NO. 3) enthalten ist:

5. DNA-Strang nach Anspruch 1, dadurch gekennzeichnet, daß mindestens ein Teil von dessen eigener Sequenz oder der Sequenz, die dazu komplementär ist, in einem Bereich außerhalb des Bereichs, der die Dinukleotid-Wiederholungen oder -Repeats dieses letzteren enthält, innerhalb der folgenden Sequenz C161 (SEQ ID NO. 4) enthalten ist:

6. DNA-Strang nach Anspruch 1, dadurch gekennzeichnet, daß mindestens ein Teil von dessen eigener Sequenz oder der Sequenz, die dazu komplementär ist, in einem Bereich außerhalb des Bereichs, der die Dinukleotid-Wiederholungen oder -Repeats dieses letzteren enthält, innerhalb der folgenden Sequenz C171 (SEQ ID NO. 5) enthalten ist:

7. Paar von Primern, insbesondere für die PCR, dadurch gekennzeichnet, daß deren eigene Sequenzen in den folgenden zwei Sequenzen enthalten sind: oder umgekehrt.

8. Paar von Primern gemäß Anspruch 7, dadurch gekennzeichnet, daß die zwei Primer jeweils die folgenden Nukleotidsequenzen aufweisen:

9. Paar von Primern, insbesondere für die PCR, dadurch gekennzeichnet, daß deren eigene Sequenzen in den folgenden zwei Sequenzen enthalten sind: oder umgekehrt.

10. Paar von Primern gemäß Anspruch 9, dadurch gekennzeichnet, daß die zwei Primer jeweils die folgenden Nukleotidsequenzen aufweisen:

11. Paar von Primern, insbesondere für die PCR, dadurch gekennzeichnet, daß deren eigene Sequenzen in den folgenden zwei Sequenzen enthalten sind: oder umgekehrt.

12. Paar von Primern gemäß Anspruch 11, dadurch gekennzeichnet, daß die zwei Primer jeweils die folgenden Nukleotidsequenzen aufweisen:

13. Paar von Primern, insbesondere für die PCR: oder umgekehrt.

14. Paar von Primern gemäß Anspruch 13, dadurch gekennzeichnet, daß die zwei Primer jeweils die folgenden Nukleotidsequenzen aufweisen:

15. Paar von Primern, insbesondere für die PCR, dadurch gekennzeichnet, daß deren eigene Sequenzen in den folgenden zwei Sequenzen enthalten sind:
**AFM268xd20**

16. Fragment von DNAs, dadurch gekennzeichnet, daß deren Sequenzen ausgewählt sind aus den vollständigen Sequenzen, die in einem der Ansprüche 2 bis 6 definiert sind, oder den Sequenzen, die zu diesen jeweils komplementär sind.

17. Fragment, das das DNA-Fragment von Anspruch 16 oder einen Teil dieses Fragments mit einem nicht-menschlichen Fragment rekombiniert.

18. Verwendung eines Fragments nach Anspruch 17 als Hybridisierungssonde für den direkten Nachweis des diesem Fragment entsprechenden Markers oder des Bereichs, der diesen enthält.

19. Verfahren zur Untersuchung des menschlichen Chromosomenbereichs 5q13, welcher mindestens eines der Gene enthält, die entweder durch ihre Anwesenheit oder durch ihre Abwesenheit in Zusammenhang mit SMA stehen, und von einem Individuum (Patient oder Foetus) stammt, das daran erkrankt ist oder eine Anfälligkeit aufweist, daran zu erkranken, wobei dieses Verfahren umfaßt, eine Probe von chromosomaler DNA dieses Individuums oder Foetus in Kontakt zu bringen mit mindestens einem der DNA-Stränge nach einem der Ansprüche 1 bis 17, insbesondere unter Amplifikationsbedingungen, welche die Verlängerung dieser Ketten von da ab, wo dieser mit entsprechenden Markern dieser chromosomalen DNA hybridisiert, in Richtung der wiederholten Sequenzen dieser Marker und darüber hinaus begünstigen, dann sich Bedingungen auszusetzen, die es ermöglichen, zwischen den erhaltenen Ketten aus der Verlängerung entsprechend ihrer jeweiligen Längen zu unterscheiden, um für die Anzahl dieser Marker repräsentative Informationen zu erhalten.

20. Verfahren nach Anspruch 19, dadurch gekennzeichnet, daß es umfaßt, die chromosomale Probe mit einem Paar von Primern entsprechend einem der Ansprüche 7 bis 15 in Kontakt zu bringen, und daß die Unterscheidung zwischen den Ketten aus der Verlängerung insbesondere durch elektrophoretische Wanderungen in einem Gel erzielt wird.
